# EUROPEAN PATENT APPLICATION

(11) **EP 1 707 578 A2**
(43) Date of publication of application: **04.10.2006**
(21) Application number: 06003092.1
(22) Date of filing: 15.02.2006
(51) Int. Cl.: C08B 37/08, A61K 31/728, A23L 1/30

(54) **Preparation of low molecular weight hyaluronic acid as a food supplement**

(30) Priority: 15.02.2005 US 57882
(71) Applicant: Collagen Nutraceuticals, Inc., Lake Forest, CA 92630 (US)
(72) Inventor: Alkayali, Ahmad, Lake Forest, CA 92630 (US)
(74) Representative: Winter, Brandl, Fürniss, Hübner Röss, Kaiser, Polte Partnerschaft Patent- und Rechtsanwaltskanzlei

(57) **Abstract**

A process for preparing hyaluronic acid primarily as a food supplement which is capable of absorption and assimilation by the human body includes desirable control of both the purity and molecular weight range of the resulting product. Chicken comb tissue is subjected to one of two disclosed processes for extracting, purifying, and controlling the molecular weight range of hyaluronic acid in solution, which is then dried and powdered to a form suitable for human consumption as a food supplement. The resulting hyaluronic acid product can also be used topically in creams or solutions for beneficial treatment of skin conditions, such as dry skin or wrinkling, for example.

## Description

### Field of the Invention

The present invention is in the field of preparing hyaluronic acid (or a salt thereof) in a substantially pure form and within a desirable molecular weight range, which is especially suitable and beneficial for oral consumption by humans as a food supplement. The inventive produce has both a desirable molecular weight range allowing it to be absorbed, assimilated, and utilized by the human body, and a purity and presentation of useful hyaluronic acid making the inventive product useful as a food supplement for humans. The hyaluronic acid product can also be utilized in creams or solutions for beneficial topical application, as an assistance in alleviating dry skin conditions, for example.

### Background of the Invention

Hyaluronic acid is a mucoid polysaccharide of biological origin, which is widely distributed in nature. For example, it is known that hyaluronic acid is present in various animal tissues such as umbilical cord, synovial fluid, vitreous humor, chicken rooster and chicken hen comb, egg shell membrane, and various connective tissues such as skin and cartilage.

Chemically, hyaluronic acid is a member of glycosaminoglycans and it is constituted by alternating and repeating units of D-glucuronic acid and N-acetyl-D-glucosamine, to form a linear chain having a molecular weight up to 1.3 x 10⁷ Daltons. Pharmaceutical, ophthalmic, and surgical uses of hyaluronic acid or of a salt thereof is widely described in the literature. For example, hyaluronic acid as a non-immunogenic substance has viscoelastic and hydrophilic properties, and it is used as an eye vitreous or joint fluid replacement or as a supportive medium in ophthalmic surgery, as disclosed for example in U.S. Pat. No. 4,141,973 of Balazs. In joint fluids, the viscous hyaluronic acid solution serves as a lubricant to provide a protective environment to the cells, and for this reason, it is used in the treatment of inflamed knee joints.

Hyaluronic acid of high molecular weight for pharmaceutical, surgical, and ophthalmic uses, for example, can be extracted and purified from various sources, including from umbilical cords, from chicken rooster combs, from chicken hen comb, or from group A and C Streptococci as disclosed for example in U.S. Pat. No. 4,141,973 of Balazs and U.S. Pat. No. 5,559,104 of Romeo et al. Production of hyaluronic acid by Streptococci was first disclosed by Forrest et al. in 1937, (J. Biol. Chem. 118, 61 (1937)) and later it was demonstrated that hyaluronic acid from animal source is identical to hyaluronic acid from microbial source. The biosynthesis of hyaluronic acid by Streptococci is disclosed for example in U.S. Pat. No. 4,897,349 of Swann et al. A number of processes are known for obtaining pharmaceutical grade hyaluronic acid or a sodium salt thereof from microbial sources. For example, U.S. Pat. No. 4,780,414 of Nimrod et al., U.S. Pat. No. 4,517,295 of Bracke et al., and U.S. Pat. No. 5,563,051 of Ellwood et al. disclose processes for obtaining hyaluronic acid by continuous fermentation of Streptococcus bacteria and then purifying hyaluronic acid thus obtained up to pharmaceutical grade.

It is believed, however, that the ability of the human body to synthesize hyaluronic acid as part of the natural body tissues decreases with advancing age. Accordingly, it is also believed that the supplementation of the diet with a form of hyaluronic acid that can be absorbed, assimilated, and utilized by the human body is increasingly more beneficial as age advances. Further, the ability of the human body to absorb and assimilate hyaluronic acid via the digestive tract is believed to rule out the use as a food supplement of many (if not all) of the known hyaluronic acid products currently available. That is, the high molecular weight of these products will result in their simply passing through the digestive system without absorption, assimilation, or digestion

Further, the described extraction and purification procedures are quite complex and result in high production costs, and in a product of a molecular weight range too high for use of the product as a food supplement for humans.

### Summary of the Invention

An object of the present invention is to obtain a hyaluronic acid (or a salt thereof) from chicken comb (either from rooster comb or from hen comb) which has sufficient purity for use as a food supplement for humans, and which also has a desired low molecular weight range, making the hyaluronic acid product available for absorption and assimilation via the digestive tract of humans for beneficial use in the human body as a food supplement.

According to one particularly preferred embodiment of the present invention, a method for providing a salt of hyaluronic acid (HA) with a desired molecular weight range includes steps of: providing a solution of extraction including HA and undesired proteins; removing a substantial fraction of said undesired proteins from said solution of extraction; decomposing the HA to a desired low molecular weight range; separating said decomposed HA; and drying the decomposed HA.

Other objects, characteristics, and advantages of the present invention will be apparent from a reading of the following detailed description to two particularly preferred exemplary embodiments of the invention.

### Brief Description of the Drawing Figures

Figure 1 provides a process flow chart illustrating the steps in a first preferred exemplary method of preparing hyaluronic acid as a food supplement from rooster comb; and

Figure 2 provides a process flow chart illustrating the steps in a second preferred exemplary method of preparing hyaluronic acid as a food supplement from hen comb.

### Detailed Description of Preferred Exemplary

### Embodiments of the Invention

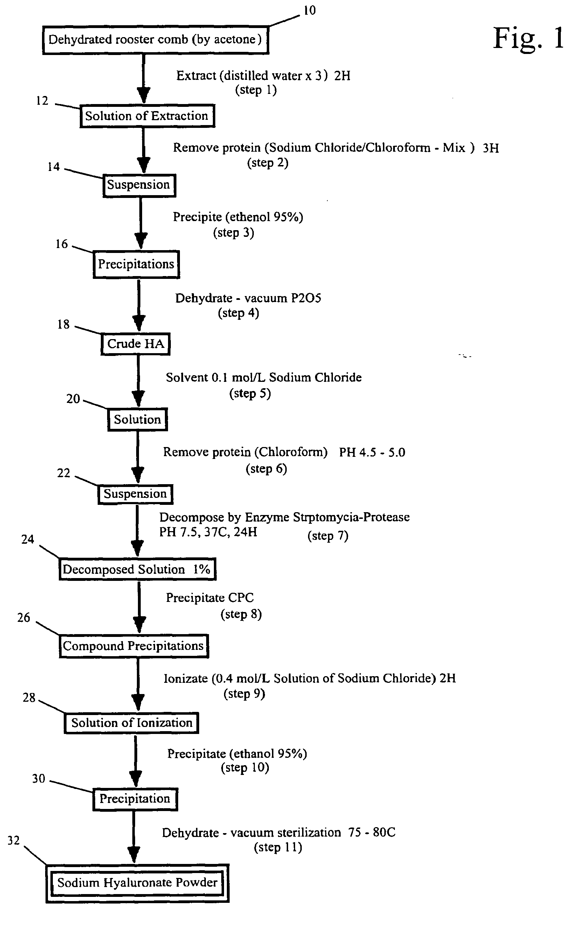

Considering first Figure 1, it is seen that a process of preparing Hyaluronic Acid (hereinafter, "HA") as a food supplement suitable for beneficial consumption by humans, according to this particularly preferred embodiment, begins (as is indicated with the numeral 10) with ground dehydrated rooster comb. That is, the raw rooster comb is harvested as part of the chicken slaughtering process, and is dehydrated using acetone and then is ground to a particle size of about 1 to 2 mm.

This dried (i.e., dehydrated) rooster comb 10 is then subjected to an extraction step (Step 1, preferably repeated three times) in which the dehydrated rooster comb is soaked in distilled water for an interval of about 2 hours. As mentioned, it is preferred that this soaking extraction step be repeated three times, although the invention is not so limited. The resulting batches of aqueous solution are combined, to produce a Solution of Extraction 12. This Solution of Extraction contains both the desired HA and undesired proteins which have also been placed into solution during the soaking step.

Undesirable proteins which have been put into solution by the soaking in distilled water are partially removed from the Solution of Extraction 12 using sodium chloride and chloroform and mixing for an interval of about 3 hours (Step 2). This step 2 results in a Suspension 14, to which ethanol is added to cause precipitation (Step 3). The resulting Precipitations 16 (separated from the solution) are dehydrated using vacuum (Step 4) to produce a Crude HA product 18 .

Next, the Crude HA product 18 is dissolved (Step 5) using a sodium chloride solvent (i.e., aqueous solution), preferably at a 0.1 mol/L concentration. This step 5 produces a Solution 20, from which proteins are removed (Step 6) by use of chloroform at a PH of about 4.5 to 5.0. This step 6 produces a suspension 22, in which the HA is decomposed to a desirably low molecular weight range (Step 7), using enzymatic digestion. That is, Strptomycia-protease enzyme is used at a PH of about 7.5, for an interval of about 24 hours, at a temperature of about 37°C, to produce a Decomposed Solution 24. As will be further explained below, the temperature and time interval of this digestion step 7 is varied in order to produce an HA product having a desired molecular weight range so that the HA product is available for absorption via the digestive tract of a human. In view of the fact that human production of HA for use in various body tissues is believed to decrease with age, it is believed also that health, flexibility of joints and elasticity of various tissues, as well as other beneficial results will be realized in the human body by the ingestion of HA in a molecular weight range as set forth herein.

The Decomposed Solution 24 will contain about 1% of HA, as is noted on Figure 1. Next, the Decomposed Solution 24 is precipitated (Step 8) and the solvent is removed to produce a Compound Precipitations 26. Next, the Compound Precipitations 26 is dissolved (Step 9) and is also ionized by use of a solvent of 0.4 mol/L sodium chloride in water, soaking the Compound Precipitations for an interval of about 2 hours.

This preceding step 9 provides a Solution of Ionization 28 which is precipitated using 95% ethanol (Step 10), and the solvent is separated, to provide a resulting Precipitation 30, which is saved. The Precipitation 30 is then dehydrated using vacuum sterilization (Step 11) at a temperature of about 75 to 80°C to produce the final Sodium Hylauronate powder 32.

Having considered the steps of a method of providing Sodium Hyaluronate powder 32, as outlined above, attention may now be given to the details of Step 7, by which a desired selective control of the molecular weight range of the product HA is exercised. That is, by selective control and variation of the time and/or temperature of the enzymatic digestion step 7, the molecular weight range of the resulting HA powder product 32 is controlled. Desirably, the HA powder 32 has a molecular weight range of from about 8,000 to about 48,000 Daltons. More desirably, the HA powder 32 has a molecular weight range of from about 8,000 to about 25,000 Daltons. Still more desirably, the HA powder 32 has a molecular weight range of from about 8,000 to about 15,000 Daltons. And, most desirably, the HA powder 32 has a molecular weight range of from about 8,000 to about 12,000 Daltons. As described above, this molecular weight range for the HA product 32 is controlled by selective control of the enzymatic digestion or decomposition step 7. That is, variations of the combination of time and temperature used for this step 7 will result in variations of the molecular weight range for the HA product 32 so that the desired molecular weight range is achieved.

Once the HA powder product is obtained, it may be packaged for distribution and use as a human food supplement. This packaging may include, for example, simply providing the powdered HA product in a bottle or jar for consumption with food or drink, or the packaging of the powder HA product into gelatin capsules for ease of swallowing, as is further explained below.

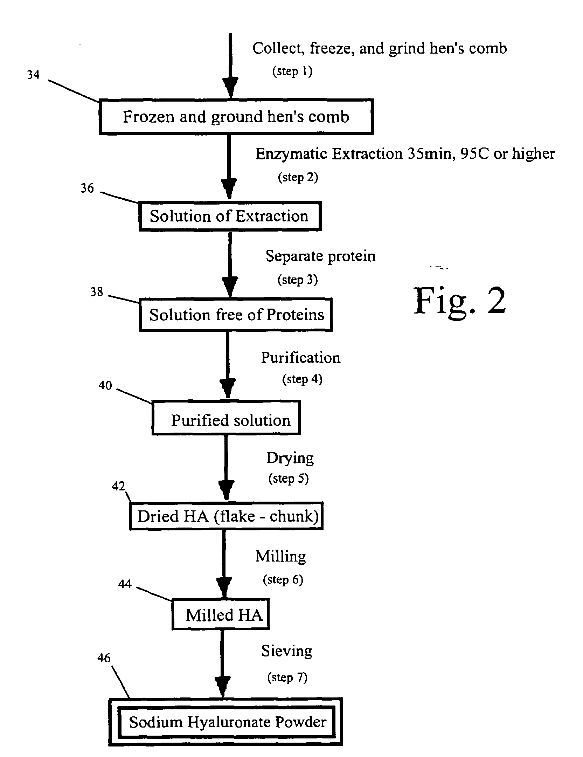

Turning now to Figure 2, an alternative method of providing a HA food supplement product is illustrated. Viewing Figure 2, it is seen that in this embodiment, the preferred starting material is chicken hen's comb. The hen's comb is collected as part of the slaughtering process, and is then frozen, and ground (step 1) to provide a collected, frozen and ground starting material 34. Most preferably, the material 34 has a granule size of from about 1 mm to about 2mm. At step 2 of this process, the starting material 34 is subjected to an enzymatic extraction step (step 2) using water as the solvent and for a time interval and at a temperature that are respectively shorter and higher than was the case with step 1 of Figure 1. However, this step 2 of the method illustrated in Figure 2 also includes an enzymatic digestion action, for which the same enzyme used in the method of Figure 1 may be used. That is, the Strptomycia-protease enzyme may be used in the water used for this extraction step. The result is a Solution of Extraction 36, which includes both the desired HA and undesired proteins also extracted from the starting material 34. However, because the Solution of Extraction 36 has been subjected to an enzymatic digestion action simultaneously with the extraction from the comb tissues, the molecular weight range of the resulting HA product will be controlled by selective variation of the time, temperature, and enzyme activity (i.e., by choice of enzyme, and enzyme concentration).

Next, the Solution of Extraction 36 is subjected to a step (step 3) to separate undesired proteins from the Solution of Extraction. As described above with reference to the method of Figure 1 (i.e., at step 6 of that method), the undesired proteins may be removed by the use of chloroform. The PH of the Solution of Extraction is controlled during the exposure to chloroform to effect the desired separation of the undesired proteins from the Solution of Extraction. The result is a Solution Free of Proteins 38. This Solution Free of Proteins is further purified (step 4) to provide a Purified Solution 40.

Then, by use of vacuum drying, a Dried HA product 42 is obtained. This product 42 will be in the form of flakes or chunks, dependent upon such factors as the particulars of the drying process, the equipment used, and the concentration of the HA in the Purified Solution 40. Next, the product 42 is subjected to milling (step 6 - milled HA is intermediate product 44) and to sieving (step 7) to provide a milled and sieved sodium hyaluronate powder 46.

Again, by selective control and variation of the time and/or temperature of the enzymatic extraction step 36, the molecular weight range of the resulting HA powder product 32 is controlled. For this process also, the HA powder 48 has a molecular weight range of from about 8,000 to about 48,000 Daltons. More desirably, the HA powder 48 has a molecular weight range of from about 8,000 to about 25,000 Daltons. Still more desirably, the HA powder 48 has a molecular weight range of from about 8,000 to about 15,000 Daltons. And, most desirably, the HA powder 48 has a molecular weight range of from about 8,000 to about 12,000 Daltons.

As described above, this molecular weight range for the HA product 48 is controlled by selective control of the enzymatic extraction step 36 That is, variations of the combination of time and temperature used for this step 36 will result in variations of the molecular weight range for the HA product 48 so that the desired molecular weight range is achieved. And again, once the HA powder product is obtained, it may be packaged for distribution and use as a human food supplement. This packaging may include, for example, simply providing the powdered HA product in a bottle or jar for consumption with food or drink, or the packaging of the powder HA product into gelatin capsules for ease of swallowing. As an example of such packaging of the HA powder into gelatin capsules, a preferred capsule size is from 50 to 100 mg capsules, with an adult daily dosage of 100 to 200mg. Alternatively, the capsule size may be from 100mg to about 150mg, with the number of capsules per day adjusted accordingly. Or, still alternatively, the capsule size may be from about 200mg to about 250mg, and the daily dosage would be one or two capsules once or twice per day for an adult human as a food supplement.

In view of the above, it will be appreciated that the present invention may be subject to many alterations and variations without departing from the spirit and scope of the appended claims, which alone define the invention. That is, the starting material for the method of Figure 1 is chicken rooster comb, while the starting material for the method of Figure 2 is chicken hen's comb. However, the method of Figure 1 could be used to extract HA from hen's comb, while the method of Figure 2 could be used to extract HA from rooster comb. In each instance a desirable HA food supplement product could be obtained, even though the particulars of the method of both Figures 1 and 2 are optimized for rooster comb and for hen's comb, respectively. Further, it will be recognized that the teachings of this invention could be used to obtain HA as a food supplement product from staring materials other than chicken comb (i.e., from materials other than rooster comb or hen's comb).

Moreover, the teachings of this invention could be used to extract a food supplement HA product from, for example, turkey comb. While the tissues of turkey comb may differ somewhat from rooster comb (e.g., possibly having a higher constituent of sodium than is the case with rooster comb), this difference in the starting material can be compensated for by using a slightly less salty concentration of sodium chloride at step 2 of the method of Figure 1, for example. By thus adjusting the particulars of the methods of Figures 1 and 2 as taught by this invention, HA as a food supplement material can be obtained from a variety of starting materials. Importantly, it will be noted that the solutions initially obtained from the starting materials according to this invention contain both extracted HA, and undesired proteins also obtained from the starting materials (i.e., from the rooster comb tissue, for example). According to the teaching of this invention, although the undesired proteins are also extracted along with the desired HA, these proteins are subsequently removed and are separated from the HA, so that a final HA food product of sufficient purity for human consumption is obtained. Also, very importantly, the HA food supplement product obtained according to the teaching of this present invention has a desirably low molecular weight range to allow its oral consumption by a human, with beneficial assimilation and utilization by the human body.

## Claims

1. A method of providing hyaluronic acid (HA) in the form of a salt of HA with a desired molecular weight range for beneficial human oral consumption as a food supplement, said method comprising steps of:
providing a solution of extraction including HA and undesired proteins;
removing a substantial fraction of said undesired proteins from said solution of extraction;
decomposing the HA to a desired low molecular weight range allowing for beneficial human oral consumption as a food supplement;
separating said decomposed HA from solution; and
drying the decomposed HA to a form for human oral consumption as a food supplement.

2. The method of Claim 1 including providing said solution of extraction by aqueous extraction from chicken comb.

3. The method of Claim 2 including the step of utilizing chicken rooster comb.

4. The method of Claim 3 further including the steps of:
utilizing dehydrated, granulated rooster comb and soaking said rooster comb in distilled water to produce said solution of extraction including both HA and undesired proteins;
removing undesired proteins from said solution of extraction by mixing with sodium chloride and chloroform to provide a suspension;
precipitating said suspension using ethanol, and removing the liquid fraction; at least partially dehydrating the solid fraction of said suspension;
re-hydrating said solid fraction using an aqueous solution of sodium chloride;
removing proteins by use of chloroform at a PH in the range of about 4.5 to about 5.0 to provide a suspension including HA;
performing enzymatic decomposition on said suspension to provide a decomposition suspension including HA having a desired molecular weight range;
precipitating said decomposition suspension to provide a compound of precipitations;
ionizing said compound of precipitations using an aqueous solution of sodium chloride to provide a solution of ionization;
precipitating said solution of ionization using ethanol to provide a precipitation; and
dehydrating said precipitation to provide dry sodium hyaluronate powder having said desired molecular weight range.

5. A dry sodium hyaluronate powder produced according to the method of Claim 4.

6. The method of Claim 4 further including the step of producing said HA with said desired molecular weight range from about 8000 Daltons to about 48,000 Daltons.

7. The method of Claim 6 further including the step of producing said HA with said desired molecular weight range from about 8,000 Daltons to about 25,000 Daltons.

8. The method of Claim 7 further including the step of producing said HA with said desired molecular weight range from about 8,000 Daltons to about 15,000 Daltons.

9. The method of Claim 8 further including the step of producing said HA with said desired molecular weight range from about 8,000 Daltons to about 12,000 Daltons.

10. The method of Claim 4 further including the step of packaging said sodium hyaluronate powder in gelatin capsules each of from about 50mg to about 250mg size.

11. The method of Claim 10 further including the step of packaging said sodium hyaluronate powder in gelatin capsules each of from about 100mg to about 200mg size.

12. The method of Claim 11 further including the step of packaging said sodium hyaluronate powder in gelatin capsules each of about 150mg size.

13. The method of Claim 2 including the step of utilizing chicken hen's comb.

14. The method of Claim 13 further including the steps of:
utilizing frozen, ground hen's comb;
hydrating said frozen, ground hen's comb using an aqueous solution including enzyme;
heating the aqueous solution including enzyme and hydrated hen's comb to a temperature and for a time sufficient to simultaneously perform both extraction of HA and undesired proteins from the hen's comb, while also effecting enzymatic decomposition of the extracted HA to a desired low molecular weight range, providing said solution of extraction;
removing undesired proteins from said solution of extraction to provide a solution substantially free of undesired proteins;
purifying said solution substantially free of undesired proteins to provide a purified solution; and
dehydrating said purified solution to provide a dried salt of HA having said desired molecular weight range.

15. The method of Claim 14 including the steps of milling said dried salt of HA, and sieving the milled dried salt of HA, to provide sodium hyaluronate powder having said desired molecular weight range.

16. A dry sodium hyaluronate powder produced according to the method of Claim 14.

17. A method of providing hyaluronic acid (HA) in the form of a salt of HA with a desired molecular weight range for beneficial human utilization, said method comprising steps of:
providing a solution of extraction including HA and undesired proteins;
removing a substantial fraction of said undesired proteins from said solution of extraction;
decomposing the HA to a desired low molecular weight range allowing for beneficial human utilization of said HA;
separating said decomposed HA from solution; and
drying the decomposed HA to a form for human utilization.

18. The method of Claim 17 further including the step of providing said HA for topical human application as part of a material selected from the group consisting of:
an aqueous serum for topical application, a cream for topical application, and a solution for topical application.

19. The method of Claim 17 further including the steps of
utilizing dehydrated, granulated rooster comb;
soaking said dehydrated, granulated rooster comb in distilled water to produce said solution of extraction including both HA and undesired proteins;
removing undesired proteins from said solution of extraction by mixing with sodium chloride and chloroform to provide a suspension;
precipitating said suspension using ethanol, and removing the liquid fraction;
at least partially dehydrating the solid fraction of said suspension;
re-hydrating said solid fraction using an aqueous solution of sodium chloride;
removing proteins from said re-hydrated solid fraction by use of chloroform at a PH in the range of about 4.5 to about 5.0 to provide a suspension including HA;
performing enzymatic decomposition on said suspension to provide a decomposition suspension including HA having a desired molecular weight range;
precipitating said decomposition suspension to provide a compound of precipitations;
ionizing said compound of precipitations using an aqueous solution of sodium chloride to provide a solution of ionization;
precipitating said solution of ionization using ethanol to provide a precipitation; and
dehydrating said precipitation to provide dry sodium hyaluronate powder having said desired molecular weight range.

20. A dry sodium hyaluronate powder produced according to the method of Claim 19.
